# FASCICULE DE BREVET EUROPEEN

(11) **EP 1 686 881 B1**
(45) Date de publication et mention de la délivrance du brevet: **28.06.2017**
(21) Numéro de dépôt: 04805420.9
(22) Date de dépôt: 09.11.2004
(51) Int. Cl.: A61K 8/37, A61Q 19/00, A61K 8/02

(54) **PRODUIT EN MATERIAU FIBREUX CONFERANT UNE SENSATION DE FRAICHEUR A SON CONTACT**
FASERPRODUKT, DAS EIN BEI KONTAKT EIN FRISCHEGEFÜHL VERLEIHT
TEXTILE PRODUCT PROVIDING A FRESH FEELING FOR A SKIN BY CONTACT THEREWITH

(30) Priorité: 14.11.2003 FR 0313344
(43) Date de publication de la demande: 09.08.2006
(73) Titulaire: SCA TISSUE FRANCE, 93400 ST-OUEN (FR)
(72) Inventeur: BRET, Bruno, F-68920 Wintzenheim (FR); JEANNOT, Sébastien, F-68320 Bischwihr (FR)
(74) Mandataire: Gevers & Orès
(86) Numéro de dépôt international: PCT/FR2004/002877
(87) Numéro de publication internationale: WO 2005/048800

(56) Documents cités:
- WO-A-97/30217
- GB-A- 1 103 040
- DATABASE WPI Derwent Publications Ltd., London, GB; AN 1979-32348B XP002288606 & JP 54 035215 A (KUSANO K) 15 mars 1979 (1979-03-15)

## Description

La présente invention concerne le domaine des matériaux fibreux, notamment cellulosiques, qui sont mis au contact de la peau en utilisation, tels que les papiers absorbants ou les produits en nappe ou en non-tissé. Pour les papiers absorbants, elle concerne notamment les produits tels que le papier toilette, les mouchoirs, serviettes, serviettes à démaquiller, chiffons, lingettes sèches ou essuie-mains. Pour les produits en nappe ou en non-tissé, les fibres sont naturelles, en particulier en coton, artificielles ou synthétiques. L'invention s'applique ainsi au coton dit hydrophile en nappe et au coton non tissé.

Elle vise en particulier de tels produits sur lesquels est appliqué un agent dont la fonction est de conférer un effet rafraîchissant, une sensation de fraîcheur, lorsque les utilisateurs les mettent au contact de leur peau.

On entend par papier absorbant l'ouate de cellulose, aussi appelée papier tissue dans le présent domaine technique, et obtenue par voie humide ou le papier obtenu par voie sèche et constitué de fibres papetières liées par un liant tel que le latex.

Comme on utilise ces produits en les frottant sur la peau, les fabricants cherchent régulièrement à en améliorer la qualité de contact et le confort d'utilisation en agissant sur le mode de fabrication de la feuille, les fibres qui les composent ou l'état de surface.

Pour le papier par exemple, un moyen consiste à appliquer une lotion en surface en plus ou moins grande quantité. La lotion améliore les qualités de douceur, de glissant et de souplesse au toucher. Elle évite que le produit ne soit irritant pour la peau. Cette qualité est recherchée, par exemple, pour des mouchoirs en papier. L'application d'une lotion appropriée sur le papier réduit l'irritation que produit le frottement du mouchoir contre le nez.

La demanderesse a développé des lotions ayant un effet émollient sur la peau, comme par exemple celle décrite dans le brevet EP 0 882 155.

Il est connu aussi d'appliquer une lotion ou un parfum sur le papier qui lui confère un effet rafraîchissant pour l'utilisateur. Par exemple la demanderesse commercialise des mouchoirs parfumés au menthol qui ont cette propriété.

Cependant, le menthol peut ne pas être accepté par l'utilisateur car son parfum a un caractère fort. La demanderesse s'est fixé comme objectif la mise au point d'un nouveau produit fibreux lotionné dont le contact avec la peau produit une sensation de fraîcheur à son utilisateur sans que celle-ci soit liée à une odeur particulière.

Conformément à l'invention, le produit en matériau fibreux conférant une sensation de fraîcheur quand il est mis au contact de la peau est caractérisé par le fait qu'il comprend au moins partiellement en surface de 2 g/m² à 6 g/m² d'un agent comprenant au moins un ou un mélange d'esters d'acide gras dont la chaîne carbonée est en C10-C14 et d'alcool dont la chaîne carbonée est en C10-C14, la température de fusion de l'agent est comprise entre 20 et 37°C.

L'agent comprend éventuellement un autre composé, par exemple choisi parmi les solvants, les esters d'acides gras, les alcools gras ou les huiles minérales. Il comprend cependant au moins 60 % en poids dudit ester.

Avantageusement, l'agent comprend l'ester dodécylique d'acide dodécanoïque ou laurate de lauryle.

Le laurate de lauryle est un ester d'acide gras de formule CH₃(CH₂)₁₀COO(CH₂)₁₁CH₃ et peut être produit à partir d'alcool laurylique et de laurate de méthyle. Un produit comprenant 75 % en poids de laurate de lauryle est commercialisé par la société STRAHL & PITSCH sous la dénomination PURESTER, en particulier la dénomination PURESTER 24. Il présente la particularité d'avoir une température de fusion autour de 25°C.

Ce produit est connu dans le domaine des produits cosmétiques et entre dans la composition de crèmes ou de lotions, en particulier de bâtonnets de rouge à lèvre par exemple, de lotions de protection solaire, d'huiles de bain ou shampooings.

L'agent contient donc avantageusement de 75 % à 40 % en poids, et préférentiellement 45 % en poids de laurate de lauryle lorsqu'il est préparé avec le PURESTER 24.

On a constaté avec surprise qu'en appliquant sur un matériau fibreux tel qu'une feuille de papier absorbant une quantité aussi faible c'est à dire en moyenne et au moins partiellement sur la surface de 2 g/m² à 6 g/m² d'un tel agent, on obtenait une sensation de fraîcheur au toucher. Le produit en comprend au moins 2 g/m² en moyenne et, en pratique, il en comprend moins de 6 g/m². De préférence, il est majoritairement en surface, une faible proportion pouvant être absorbée par le substrat.

L'agent peut être appliqué sur la surface en continu, mais de préférence il est appliqué de manière discontinue de manière à avoir des quantités plus élevées localement, par exemple le long de bandes parallèles ou sous la forme de pavés répartis uniformément ou non sur la surface.

On décrit ci-après plusieurs modes d'application de l'agent sur une feuille de papier tissue en référence aux dessins sur lesquels :
- la figure 1 montre un premier mode d'application par dépôt au moyen d'un bac pourvu d'une buse à lèvres,
- la figure 2 montre un deuxième mode d'application par rouleau. L'installation représentée sur la figure 1 est de type à extrusion sur une feuille en mouvement.

Le traitement peut être appliqué en cours de transformation d'une feuille issue d'une bobine mère. Par exemple pour un papier absorbant, sa transformation en papier toilette comprend une étape de gaufrage dans une installation de gaufrage et d'assemblage, le cas échéant, de plusieurs plis les uns sur les autres pour former une feuille multi-plis. La feuille ainsi transformée est ensuite guidée vers un poste de bobinage et de découpe pour la mise en rouleaux individuels.

Dans cette application, le traitement de l'invention est avantageusement effectué sur le parcours de la feuille entre l'étape de gaufrage et celle de mise en rouleau. Cependant, le traitement peut aussi bien être appliqué sur une feuille avant gaufrage.

La feuille 1 est entraînée jusqu'à une station d'application de l'agent. Cette station comprend un bac 10 disposé transversalement par rapport au sens de défilement de la feuille à traiter. Le fond du bac est ouvert le long d'une fente 12 avec deux bords parallèles 11 et 13 formant une buse à lèvres. Pour une pression et un état de fluide donnés, la largeur de la fente 12 et la distance entre les lèvres déterminent le débit de liquide qui est déposé sur la feuille. Au lieu d'une seule fente, on peut ménager plusieurs fentes sur la largeur de la laize. Chaque fente détermine la largeur d'une bande de produit déposé sur la feuille de papier.

En choisissant des fentes de largeur appropriée, on assure le dépôt soit sur une surface continue en travers de la laize soit sur des surfaces distinctes en forme de bandes. On peut également déposer l'agent sur des portions de bandes successives en interrompant périodiquement l'alimentation 14 du bac 10 qui délivre l'agent à l'état fluide à la ou aux buses à lèvre.

Le bac 10 comprend un moyen de chauffage permettant de maintenir l'agent à la température choisie. Dans le cas d'un agent composé d'au moins 75% en poids de laurate de lauryle, la température de fusion étant de 25°C, on maintient la température dans le bac à 25°C au moins sans toutefois dépasser une température où sa fluidité serait telle que le papier en serait trop imprégné. De préférence, on l'applique en surface du papier.

On a procédé à des essais d'application de l'agent sur du papier tissue au moyen d'un équipement avec buse à lèvres fourni par la société NORDSON. L'équipement était placé dans une installation de transformation entre l'unité de gaufrage et la station de mise en rouleaux.

Plusieurs largeurs de buses d'application ont été essayées. Des buses ayant des ouvertures respectivement de 11 mm, 22 mm, 70 mm espacées respectivement de 22 mm, 22 mm et 40 mm, et une buse sur toute la largeur de la laize.

On a maintenu la température de l'agent, PURESTER 24® entre 37 et 50°C au moment de son dépôt sur la feuille. Le taux d'application a varié entre 0,1 et 10 g/m² par bande.

Les échantillons produits ont été soumis à un panel d'utilisateurs. Un effet rafraîchissant a été constaté à partir d'une quantité moyenne appliquée de 2 g/m² en moyenne. Il était jugé satisfaisant à partir de 3 g/m².

D'autres modes d'application sont possibles.

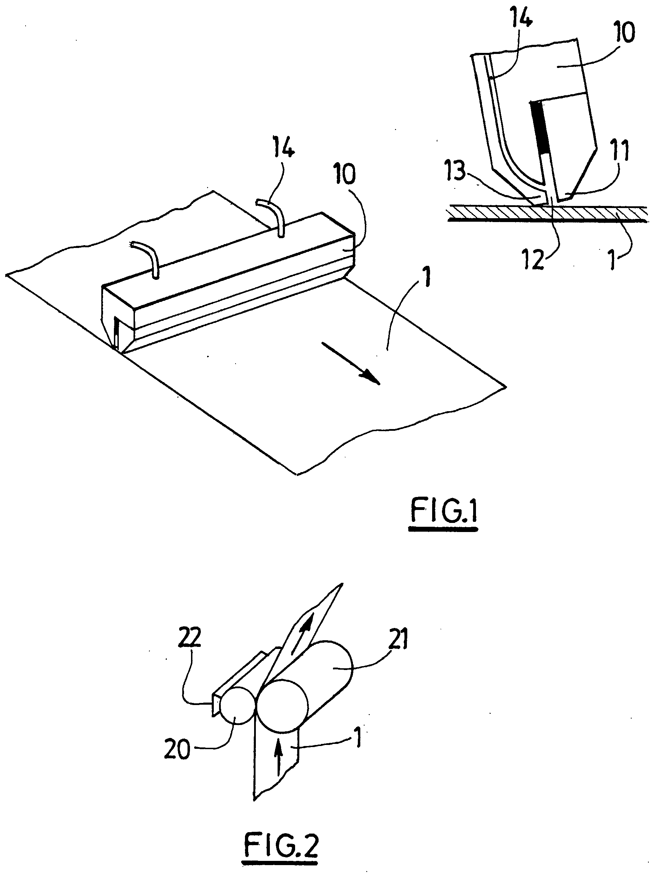

La technique d'enduction par rouleau telle que représentée sur la figure 2 consiste à faire passer la feuille 1 de produit fibreux entre un rouleau applicateur 20 et un contre rouleau 21. Le rouleau applicateur comporte des alvéoles contenant l'agent à l'état liquide et alimentées par une boîte à racles 22. L'ensemble est maintenu à une température suffisante pour que l'agent reste à l'état fluide en étant déposé sur la feuille au moment où celle-ci passe dans l'intervalle ménagé entre les deux rouleaux 20 et 21. Le dépôt en quantités dosées peut être continu ou bien discontinu selon la gravure du rouleau applicateur.

Dans une variante non représentée, on applique l'agent par le moyen d'un cylindre de transfert entre le cylindre comportant les alvéoles et le contre rouleau.

Selon une autre technique non représentée, on applique l'agent par pulvérisation par exemple selon la technique décrite dans le brevet déposé au nom de la demanderesse EP 1 108 814.

## Revendications

1. Produit en matériau fibreux **caractérisé par le fait qu'**il comporte au moins partiellement en surface de 2 g/m² à 6 g/m² d'un agent comprenant un ou un mélange d'esters d'acide gras dont la chaîne carbonée est en C₁₀-C₁₄ et d'alcool dont la chaîne carbonée est en C₁₀-C₁₄, la température de fusion de l'agent étant comprise entre 20 et 37°C.

2. Produit en matériau fibreux selon la revendication précédente dont l'agent comprend au moins 60% en poids du ou desdits esters, le reste étant choisi parmi les solvants, les esters d'acide gras, les alcools gras ou les huiles minérales.

3. Produit en matériau fibreux selon l'une des revendications précédentes dont l'agent comprend au moins l'ester dodécylique d'acide dodécanoïque

4. Produit selon la revendication précédente dont l'agent comprend entre 75% et 40 % en poids de l'ester dodécylique d'acide dodécanoïque.

5. Produit selon l'une des revendications précédentes dont l'agent est réparti majoritairement en surface.

6. Produit selon l'une des revendications précédentes dont l'agent est réparti de façon discontinue.

7. Produit selon la revendication précédente dont l'agent est réparti sous formes de bandes parallèles entre elles.

8. Produit selon l'une des revendications 1 à 5 dont l'agent est réparti sous la forme d'un film continu.

9. Produit selon l'une des revendications précédentes dont l'agent est réparti sur les deux surfaces.

10. Produit selon l'une des revendications précédentes dont le matériau fibreux comprend un papier absorbant tel que l'ouate de cellulose ou le papier voie sèche.

11. Produit selon l'une des revendications 1 à 9, dont le matériau fibreux comprend une nappe ou un non-tissé de fibres textiles naturelles, artificielles ou synthétiques.

12. Produit selon la revendication précédente dont le matériau fibreux est en coton éventuellement en mélange avec des fibres artificielles ou synthétiques.

## Patentansprüche

1. Produkt aus Fasermaterial, **dadurch gekennzeichnet, dass** es mindestens teilweise auf der Oberfläche von 2 g/m² bis 6 g/m² eines Mittels aufweist, umfassend eines von oder eine Mischung aus Fettsäureestern, deren Kohlenwasserstoffkette C₁₀-C₁₄ aufweist, und Alkohol, dessen Kohlenwasserstoffkette C₁₀-C₁₄ aufweist, wobei die Fusionstemperatur des Mittels zwischen 20 und 37 °C liegt.

2. Produkt aus Fasermaterial nach dem vorhergehenden Anspruch, dessen Mittel mindestens 60 Gew.% des oder der Ester umfasst, wobei der Rest ausgewählt ist aus den Lösemitteln, den Fettsäureestern, den Fettalkoholen oder den Mineralölen.

3. Produkt aus Fasermaterial nach einem der vorhergehenden Ansprüche, dessen Mittel mindestens Dodekansäuredodecylester umfasst.

4. Produkt nach dem vorhergehenden Anspruch, dessen Mittel zwischen 75 Gew.% und 40 Gew.% des Dodekansäuredodecylesters umfasst.

5. Produkt nach einem der vorhergehenden Ansprüche, dessen Mittel überwiegend auf der Oberfläche verteilt ist.

6. Produkt nach einem der vorhergehenden Ansprüche, dessen Mittel auf unterbrochene Weise verteilt ist.

7. Produkt nach dem vorhergehenden Anspruch, dessen Mittel in Form von untereinander parallelen Bändern verteilt ist.

8. Produkt nach einem der Ansprüche 1 bis 5, dessen Mittel in Form eines ununterbrochenen Films verteilt ist.

9. Produkt nach einem der vorhergehenden Ansprüche, dessen Mittel auf den zwei Oberflächen verteilt ist.

10. Produkt nach einem der vorhergehenden Ansprüche, dessen Fasermaterial ein absorbierendes Papier wie z. B. Zellstoffwatte oder Trockenwegpapier umfasst.

11. Produkt nach einem der Ansprüche 1 bis 9, dessen Fasermaterial eine Lage oder ein Vlies aus natürlichen, künstlichen oder synthetischen Textilfasern umfasst.

12. Produkt nach dem vorhergehenden Anspruch, dessen Fasermaterial aus Baumwolle, eventuell in Mischung mit künstlichen oder synthetischen Fasern ist.

## Claims

1. Product made of fibrous material, **characterised by** the fact that it comprises, at least partially on the surface, from 2g/m² to 6g/m² of an agent comprising one or a mixture of fatty acid esters having a C₁₀-C₁₄ carbon chain and of alcohol having a C₁₀-C₁₄ carbon chain, the melting temperature of the agent being between 20 and 37 °C.

2. Product made of fibrous material according to the preceding claim, the agent of which comprises at least 60%, by weight, of said ester or esters, the rest being chosen from solvents, fatty acid esters, fatty alcohols or mineral oils.

3. Product made of fibrous material according to one of the preceding claims, the agent of which comprises at least the dodecyl ester of dodecanoic acid.

4. Product according to the preceding claim, the agent of which comprises between 75% and 40%, by weight, of the dodecyl ester of dodecanoic acid.

5. Product according to one of the preceding claims, the agent of which is spread out mostly on the surface.

6. Product according to one of the preceding claims, the agent of which is spread out discontinuously.

7. Product according to the preceding claim, the agent of which is spread out in the form of parallel strips.

8. Product according to one claims 1 to 5, the agent of which is spread out in the form of a continuous film.

9. Product according to one of the preceding claims, the agent of which is spread out on both surfaces.

10. Product according to one of the preceding claims, the fibrous material of which comprises an absorbent paper such as cellulose wadding or dry-process paper.

11. Product according to one of claims 1 to 9, the fibrous material of which comprises a fabric or a nonwoven fabric of natural, artificial or synthetic textile fibres.

12. Product according to the preceding claim, the fibrous material of which is made of cotton optionally mixed with artificial or synthetic fibres.
